(19) Europäisches Patentamt
European Patent Office
Office européen des brevets

(11) Publication number: **0 518 458 A1**

# (12) EUROPEAN PATENT APPLICATION

(21) Application number: **92300232.3**

(51) Int. Cl.5: **C07D 233/58**, A01N 43/50

(22) Date of filing: **10.01.92**

(30) Priority: **10.06.91 US 712756**

(43) Date of publication of application:
**16.12.92 Bulletin 92/51**

(84) Designated Contracting States:
**DE FR GB**

(71) Applicant: **TEXACO CHEMICAL COMPANY**
**3040 Post Oak Boulevard**
**Houston, Texas 77056(US)**

(72) Inventor: **Su, Wei-Yang**
**11814 Knights Bridge**
**Austin, Texas 78759(US)**
Inventor: **Naylor, Carter Graham**
**8103 Forest Mesa Drive**
**Austin, Texas 78759(US)**

(74) Representative: **Brock, Peter William et al**
**UROUHART-DYKES & LORD 91 Wimpole Street**
**London W1M 8AH(GB)**

## (54) Fatty imidazolium salts.

(57) Fatty imidazolium salts which are useful as algicides have the formula:

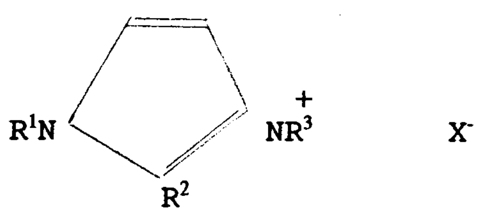

wherein $R^1$ is an alkyl group having 1 to 6 carbon atoms, $R^2$ is a fatty alkyl group having 8 to 36 carbon atoms, or an alkylene group having 8 to 36 carbon atoms substituted by an imidazolium ring of the formula:

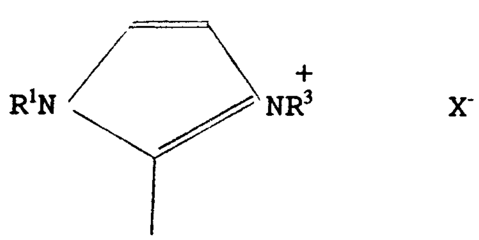

$R^3$ is an alkyl or aralkyl group having 1 to 8 carbon atoms, and X is a halide or methylsulphate anion.

The imidazolium salts can be prepared by

(a) reacting a fatty acid and an N-alkylethylenediamine

(b) dehydrogenating the resulting reaction product to form a fatty imidazole, and

(c) reacting the fatty imidazole with an aliphatic or araliphatic halide or dimethyl sulphate at a temperature of 70 to 120°C and a pressure of 0.1 to 21 MPa.

EP 0 518 458 A1

This invention relates to novel fatty imidazolium salts and more particularly this invention relates to fatty imidazolium halide and methylsulphate salts.

The novel fatty imidazolium salts are useful as algicides, exhibiting higher activity against algae than commercially available swimming pool algicides.

This invention is related to fatty imidazolium salts which are useful as algicides. The novel fatty imidazolium salts of the present invention differ from those of related art in the number of ways. Among other differences, they have a long chain alkyl group on the 2-position, and another alkyl group without other functional groups on the 1-position. They exhibit a lower degree of foaming and a higher activity against algae.

US-A-4017631 discloses quaternary imidazolium salts in which one nitrogen of the imidazolium cation is substituted with a

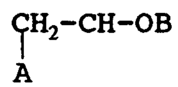

group in which A is an aryl group and B is an aliphatic or aromatic group. These salts have the formula:

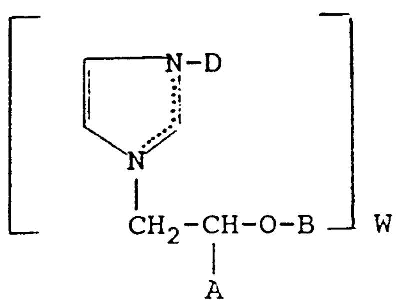

(I)

and are useful as antimicrobial agents.

DE-A-2531031 describes the preparation of imidazolium salts of the formula:

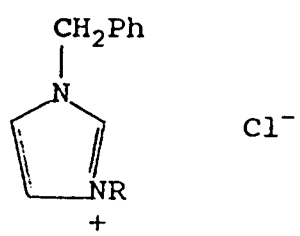

(II)

useful as non-foaming bactericides and fungicides which are the products of the reaction of imidazole with $PhCH_2Cl$ in EtOH.

US-A-3991202 also describes and teaches the preparation of a quaternary imidazolium salt represented by formula (I) above. The group represented by D is different, but the products are also useful as antimicrobial agents.

FR-A-1468184 describes a substituted benzylimidazole having the general formula:

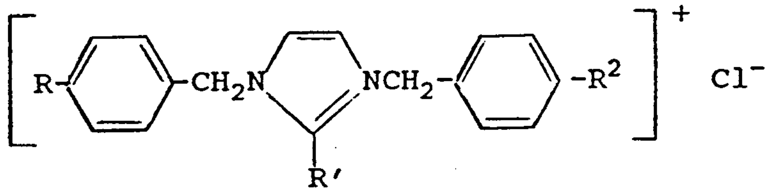

(III)

formed by treating substituted benzylimidazoles with alkylbenzyl compounds. The compounds are useful as

algicides, bactericides and fungicides.

Compounds of the formula:

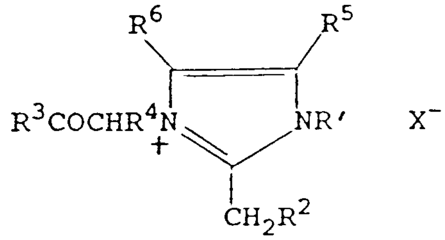

(IV)

are formed by the reaction of 1,2-dialkylimidazoles with $\beta$-haloketones in work reported in Khim. Geterotsikl. Soedin (1967), 3, 527.

There is always room in the art for additional compositions which are useful as fungicides, algicides and antimicrobial agents. If, for instance, an algicide could be produced which exhibited increased activity against algae, those skilled in the art would find a number of commercial uses for such a compound, including use in swimming pool chemicals.

It would be very desirable for economic reasons if a composition exhibiting improved activity as an algicide could be produced from readily available chemicals. Another commercially attractive property for environmental reasons, would be an algicide which exhibited low-foaming in water solutions.

The present invention provides a method for preparing imidazolium salts which comprises:

(a) reacting a fatty acid and an N-alkylethylenediamine

(b) dehydrogenating the resulting reaction product to form a fatty imidazole, and

(c) reacting the fatty imidazole with an aliphatic or araliphatic halide or dimethyl sulphate at a temperature of 70 to 120 ° C and a pressure of 0.1 to 21 MPa.

The present invention also provides novel fatty imidazolium salts exhibiting activity against algae higher than a known commercial algicide.

The novel salts according to the invention have the formula:

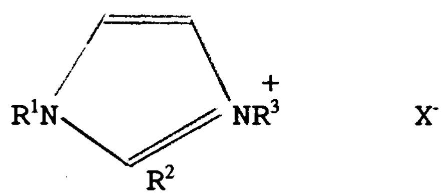

wherein $R^1$ is an alkyl group having 1 to 6 carbon atoms, $R^2$ is a fatty alkyl group having 8 to 36 carbon atoms, or an alkylene group having 8 to 36 carbon atoms substituted by an imidazolium ring of the formula:

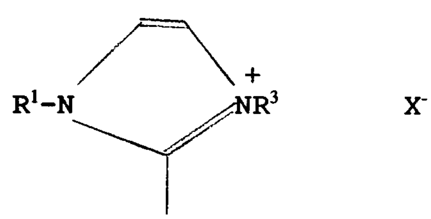

$R^3$ is an alkyl or aralkyl group having 1 to 8 carbon atoms, and X is a halide or methylsulphate anion.

The fatty imidazolium salts provide low-foaming water solutions and a high level of activity against algae.

The method for producing the novel fatty imidazolium salts of the present invention can be represented by the following:

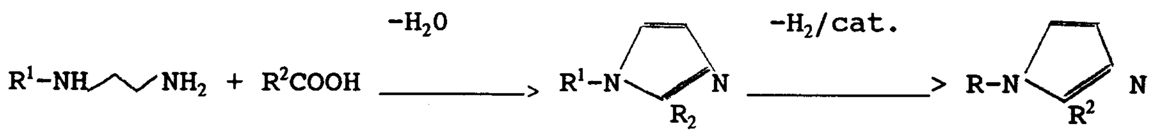


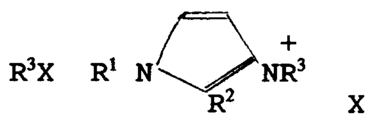

(I)

wherein $R^1$ is an alkyl group containing 1 to 6 carbon atoms, $R^2$ is a fatty alkyl group containing 8 to 36, preferably 8 to 20 carbon atoms, or an alkylene group substituted with another imidazolium ring, $R^3$ is an alkyl or aralkyl group containing 1 to 8 carbon atoms, and X is a halide or methyl sulphate ion.

Suitable fatty acids initially reacted to produce the fatty imidazole are generally those containing 8 to 36 carbon atoms. Examples include coconut acid, oleic acid, EMPOL® 1010 dimer acid, tallow acid, lauric acid, tall oil acid and stearic acid. "Dimer Acid" is dimerized polyunsaturated $C_{18}$ fatty acid formed via the Diels-Alder reaction. (See: Fatty Acids in Industry, Johnson, R.W. and Fritz, E., Ed., Martel Dekker, Inc., 1988, Ch. 7). EMPOL® is a trademark for dimer acids, 36-carbon aliphatic dibasic acids used as modifiers of synthetic polymers, soaps, corrosion inhibitors, etc., produced by Emery Industries, Inc. Coconut acid and oleic acid are very useful, as demonstrated by Examples 1 to 10.

As stated the fatty acids are reacted with N-alkylethylenediamines, for example, N-4'-methyl-2'-pentylethylenediamine,N-2'-butylethylenediamene, N-methylethylenediamine, N-isopropylethylenediamine, or N-3'-pentylethylenediamine.

The fatty acid and N-alkylethylenediamine are reacted to form fatty imidazoline intermediates, which are then dehydrogentated to form the corresponding imidazole according to procedures which are known in the art (US-A-4921969).

Subsequently the fatty imidazoles are reacted noncatalytically with dimethyl sulphate or aliphatic halides. Suitable aliphatic halides are those containing 1 to 8 carbon atoms.

Examples of suitable aliphatic halides include methyl chloride, benzyl chloride, ethyl chloride, n-butyl chloride, ethyl bromide, methyl bromide, ethyl iodide and methyl iodide.

A solvent is necessary to carry out the reaction. The preferred solvents are alcohols such as methanol, ethanol, isopropanol and t-butyl alcohol.

Reaction conditions are generally mild, but can vary according to the reacting halides. The process can be adapted so the reaction can be run under liquid phase conditions. Generally, the temperature is from 50 to 140 ° C. A narrow range of operability of 70 to 120 ° C is preferred.

Pressures of 0.1 to 21 MPa can be used.

Specific products described in the examples include;
1-isopropyl-2-cocoimidazole
1-isopropyl-2-coco-3-methylimidazolium chloride
1-isopropyl-2-oleylimidazole
1-isopropyl-2-oleyl-3-methylimidazolium chloride
1-3'-pentyl-2-cocoimidazole
1-3'-pentyl-2-coco-3-methylimidazolium chloride
Bisimidazole derived from dimerized fatty acid
Bisimidazolium chloride from dimerized fatty acid
1-isopropyl-2-coco-3-benzyl-imidazolium chloride.

The fatty imidazolium salts have been tested as algicides in comparison with a commercial algicide product and found to exhibit increased activity against algae. In addition they exhibit desirable homogeneous low-foaming properties in water solutions.

The products can be produced in a stainless steel autoclave or glass reactor. Operating conditions can be adjusted to optimize the formation of the desired fatty imidazolium salts.

The products are recovered after removing the volatiles under reduced pressure.

The products have been identified by one or more of the following analytical procedures: infrared (IR) and nuclear magnetic resonance (nmr) or a combination of these techniques. Analyses have, for the most part, been by parts in weight; all temperatures are in degrees celsius.

**EXAMPLE 1**

**Preparation of 1-Isopropyl-2-cocoimidazole**

A one-litre three-necked flask equipped with thermometer, Dean-Stark trap, stirrer and nitrogen inlet was charged with N-isopropylethylenediamine (160g, 1.57 mol) and coconut acid (320g, 1.50 mol). The mixture was heated at 150° C for 2 hours, at 200° C for 3 hours and then at 260° C for 3 hours. The water generated, and the excess of N-isopropylethylenediamine, were removed through the Dean-Stark trap. The reaction mixture was cooled to ambient temperature, 50g of nickel catalyst was added and the mixture was then heated at 200-205° C for 5 hours. The resulting reaction mixture was filtered and was a light-brown liquid (379g). The product was confirmed by its NMR spectrum to be 1-isopropyl-2-cocoimidazole.

**EXAMPLE 2**

**Preparation of 1-Isopropyl-2-coco-3-methylimidazolium Chloride**

A 300 ml stirring autoclave was charged with 60g of 1-isopropyl-2-cocoimidazoleand 50g of isopropanol. The reactor was sealed, purged of air with nitrogen, and 25g of methyl chloride was added. The reaction mixture was heated at 120° C for three hours. The volatile materials of the resulting reaction mixture were removed under reduced pressure in a rotary evaporator. A dark-green gel-like product was obtained in quantitative yield, and was confirmed by its NMR spectrum to be 1-isopropyl-2-coco-3-methylimidazolium chloride.

**EXAMPLE 3**

**Preparation of 1-Isopropyl-2-oleylimidazole**

The procedure of Example 1 was followed, except that 180g of N-isopropylethylendiamine and 423g of oleic acid were used. The resulting product (383g) was a light-brown liquid. The product was confirmed by its NNR spectrum to be 1-isopropyl-2-oleylimidazole.

**EXAMPLE 4**

**Preparation of 1-Isopropyl-2-oleyl-3-methylimidazolium Chloride**

The procedure of Example 2 was followed, except that 20g of methyl chloride, 60g of 1-isopropyl-2-oleylimidazole and 50g of isopropanol were used. A dark-green gel-like product was obtained in quantitative yield and was confirmed by its NMR spectrum to be 1-isopropyl-2-oleyl-3-methylimidazolium chloride.

**EXAMPLE 5**

**Preparation of 1-3'-Pentyl-2-cocoimidazole**

The procedure of Example 1 was followed, except that 220g of N-3'-pentylethylenediamine and 320g of coconut acid were used. The resulting product (370g) was a light-brown liquid.

The product was 1-3'-pentyl-2-cocoimidazole.

**EXAMPLE 6**

**Preparation of 1-3'-Pentyl-2-coco-3-methylimidazolium Chloride**

The procedure of Example 2 was followed, except that 16g of methyl chloride, 61g of 1-3'-pentyl-2-cocoimidaozole and 60g of isopropanol were used. A dark-green viscous liquid product was obtained in quantitative yield, and was confirmed by its NMR spectrum to be 1-3'-pentyl-2-coco-3-methylimidazolium chloride.

**EXAMPLE 7**

**Preparation of Bisimidazole from Dimer Acid**

The procedure of Example 1 was followed, except that 285g of EMPOL® 1010 dimer acid and 124.9g of N-isopropylethylenediamine were used. The resulting product was a light-brown liquid, which was confirmed to be the bisimidazole of dimer acid.

**EXAMPLE 8**

**Preparation of Bisimidazolium Chloride**

The procedure of Example 2 was followed, except that 20g of methyl chloride, 60g of the bisimidazole of Example 7 and 50g of isopropanol were used. A dark-green gel-like product was obtained in quantitative yield, and was confirmed to be the bisimidazolium chloride.

**EXAMPLE 9**

**Preparation of 1-Isopropyl-2-coco-3-benzyl-imidazolium Chloride**

A 250 ml three-necked flask equipped with a thermometer, stirrer, condenser and nitrogen inlet was charged with 83.1g of 1-isopropyl-2-cocoimidazole (Example 1), 38g of benzyl chloride and 50g of isopropanol. The mixture was heated under reflux for 3 hours. 1-Isopropyl-2-coco-3-benzylimidazolium chloride was obtained in quantitative yield.

**EXAMPLE 10**

**Usage (Algicide)**

Samples from Examples 2, 4 and 8 were tested as algicides against commercial product BAQUA CHECK® 50 (ICI). Water from the San Marcos River in San Marcos, Texas was used. The tests were conducted at ambient temperature. Samples were exposed to the sunlight under air in the laboratory. The results are listed in Table 1. BAQUA CHECK® 50 is a swimming pool algicide and has the following components.

| | | |
|---|---|---|
| $C_{14-16}N^+(Me)_2Bz$ | $C1^-$ | 42 wt% |
| $(C_{14-16})_2NMe_2Bz$ | $C1^-$ | 8 wt% |

TABLE 1

| Entry | Algicide | Conc. (ppm) | 14 Days | 20 Days | 22 Days | 24 Days | 26 Days |
|---|---|---|---|---|---|---|---|
| 1 | Example 2 | 1 | - | - | - | - | - |
| 2 | Example 2 | 3 | - | - | - | - | - |
| 3 | Example 2 | 10 | - | - | - | - | - |
| 4 | Example 4 | 1 | - | - | - | - | - |
| 5 | Example 4 | 3 | - | - | - | - | - |
| 6 | Example 4 | 10 | - | - | - | - | - |
| 7 | Example 8 | 1 | - | - | + | + | + |
| 8 | Example 8 | 3 | - | - | - | - | - |
| 9 | Example 8 | 10 | - | - | - | - | - |
| 10 | Baqua check 50 | 1 | - | + | + | + | + |
| 11 | Baqua Check 50 | 3 | - | - | + | + | + |
| 12 | Baqua Check 50 | 10 | - | - | - | - | + |
| 13 | None | - | + | + | + | + | + |

Key:
(-) growth of algae was not apparent by visual observation.
(+) growth of algae was apparent by visual observation.

## Claims

1. A method for preparing imidazolium salts which comprises:
   (a) reacting a fatty acid and an N-alkylethylenediamine
   (b) dehydrogenating the resulting reaction product to form a fatty imidazole, and
   (c) reacting the fatty imidazole with an aliphatic or araliphatic halide or dimethyl sulphate at a temperature of 70 to 120 ° C and a pressure of 0.1 to 21 MPa.

2. A method according to Claim 1 characterized in that the fatty acid is $C_{36}$ dimer acid.

3. A method according to Claim 1 characterised in that the fatty acid has 8 to 36 carbon atoms.

4. A method according to Claim 3 characterized in that the fatty acid is coconut acid or oleic acid.

5. A method according to any one of Claims 1 to 4 characterized in that the N-alkylethylenediamine has the formula :

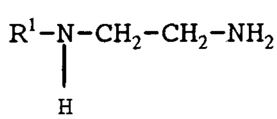

wherein $R^1$ is an alkyl group containing 1 to 6 carbon atoms.

6. A method according to Claim 5 characterized in that the N-alkylethylenediamine is N-isopropylethylenediamine or N-3'-pentylethylenediamine.

7. A method according to any one of Claims 1 to 6 characterised in that the aliphatic or araliphatic halide

has 1 to 8 carbon atoms.

**8.** A method according to Claim 7 characterised in that the halide is methyl chloride or benzyl chloride.

**9.** A fatty imidazolium salt of the formula:

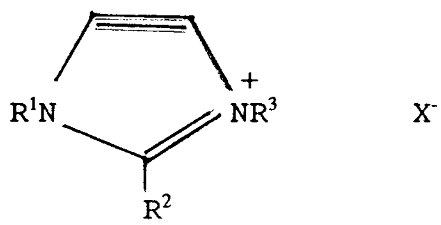

wherein $R^1$ is an alkyl group having 1 to 6 carbon atoms, $R^2$ is a fatty alkyl group having 8 to 36 carbon atoms, or an alkylene group having 8 to 36 carbon atoms substituted by an imidazolium ring of the formula:

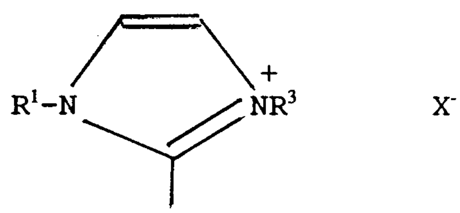

$R^3$ is an alkyl or aralkyl group having 1 to 8 carbon atoms, and $X^-$ is a halide or methylsulphate anion.

**10.** Use of a fatty imidazolium salt according to Claim 9 as an algicide.

European Patent Office

# EUROPEAN SEARCH REPORT

Application Number

EP 92 30 0232

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 493 318 (H.A. SHONLE ET AL.)<br>* examples 1-7,15 * | 1,3,9 | C07D233/58<br>A01N43/50 |
| X | US-A-2 493 319 (H.A. SHONLE ET AL.)<br>* examples 1-3,9,12 * | 1,3,9 | |
| A,D | FR-A-1 468 184 (BASF AG)<br>* the whole document * | 9,10 | |
| A | DE-B-2 510 525 (TH. GOLDSCHMIDT AG)<br>* claims 1,3 * | 9 | |
| A | FR-A-2 302 301 (TH. GOLDSCHMIDT AG)<br>* claims 1,3 * | 9 | |
| A,D | DE-B-2 531 031 (SCHÜLKE UND MAYR GMBH)<br>* claims * | 9 | |
| A,D | US-A-3 991 202 (P.A.J. JANSSEN ET AL.)<br>* claims 1,4,7 * | 9 | |
| D | & US-A-4 017 631 | | TECHNICAL FIELDS SEARCHED (Int. Cl.5)<br>C07D<br>A01N |

The present search report has been drawn up for all claims

| Place of search | Date of completion of the search | Examiner |
|---|---|---|
| BERLIN | 27 MAY 1992 | Christian Hass |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)